Europäisches Patentamt

European Patent Office

Office européen des brevets

(19)

(11) EP 0 771 869 A1

(12) **EUROPEAN PATENT APPLICATION**

(43) Date of publication:
07.05.1997 Bulletin 1997/19

(51) Int Cl.⁶: **C12N 9/64**

(21) Application number: 96307916.5

(22) Date of filing: 31.10.1996

(84) Designated Contracting States:
CH DE FR GB LI

(30) Priority: 06.11.1995 JP 309692/95

(71) Applicants:
• Hamajima, Fusanori
  Nerima-ku, Tokyo 179 (JP)
• Yamakami, Kazuo
  Wako-shi, Saitama 351-01 (JP)

(72) Inventors:
• Hamajima, Fusanori
  Nerima-ku, Tokyo 179 (JP)
• Yamakami, Kazuo
  Wako-shi, Saitama 351-01 (JP)

(74) Representative: Woods, Geoffrey Corlett
J.A. KEMP & CO.
14 South Square
Gray's Inn
London WC1R 5LX (GB)

(54) **Immunological tolerance inducing agent**

(57) An SH-dependent protease is disclosed which is obtainable by crushing placentae of a mammal to thereby obtain a homogenate, centrifuging the homogenate and subjecting the resultant supernatant to affinity chromatography (I), ion exchange chromatography, gel filtration chromatography and affinity chromatography (II) in this order and which has the following physico-chemical properties.

Function: cleaving L-prolyl-L-phenylalanyl-L-arginine 4-methyl-coumaryl-7-amide to produce L-prolyl-L-phenylalanyl-L-arginine and 7-amino-4-methyl-coumarin; Optimum pH range: 5.6 - 6.5; Range of optimum temperature: 36.5 - 37.5 °C; Stability: retaining its activity at -80 °C for at least 30 days in 5 mM acetate buffer (pH 5.0), and being completely denatured on heating at 100°C for 3 minutes; Molecular weight: 30 kDa.

An immunological tolerance inducing-agent comprising the SH-dependent protease as an active ingredient is also provided.

EP 0 771 869 A1

**Description**

BACKGROUND OF THE INVENTION

1. Field of the Invention

The present invention relates to a novel SH-dependent protease and an immunological tolerance inducing-agent comprising the protease as an active ingredient.

2. Description of the Prior Art

As immunosuppressants used for medical purposes, azathioprine, cyclosporin A, FK-506, 15-deoxyspergualin and the like are known at present and all of them are powerful in immunosuppressive effect [Murray et al., New Engl. J. Med. 268:1315 (1963); Lems & Koene, IRCS Medical Science 7:184 (1979); Inamura et al., Transplantation 45:206 (1988); and Dickneite et al., Transplantation Proceedings 19: 4244 (1987)].

However, any of these conventional immunosuppressants does not induce immunological tolerance sufficiently. Therefore, continuous administration of such an agent is required for a long time in order to achieve immunosuppression. As a result, those immunosuppressants lead to a problem of side effects, such as renal tubular disorders, angiitis, hepatopathy, pancreatic exocrine disorders and digestive tract disorders.

Under circumstances, the development of an immunosuppressant which can solve the above problem is desired.

Cysteine protease is a general term for those proteases in which an SH group is present at their active centers, and this protease is known to have an immunosuppressive action [Hamajima et al., Parasite Immunology 16:261 (1994)]. However, when a cysteine protease has been derived from a parasite, the protease will be a foreign substance to humans and other mammals and thus the protease might possibly cause side effects or the like if used as an immunosuppressant. Therefore, the use of such a cysteine protease is not necessarily desirable. On the other hand, since mammal-derived cysteine proteases hereditarily resemble with each other among mammals, it is considered that antibodies will hardly be produced and that less side effect will be caused if a mammal-derived cysteine proteases has been injected into humans or other mammals.

OBJECTS AND SUMMARY OF THE INVENTION

It is an object of the invention to provide a novel SH-dependent protease purified from a mammal and an immunological tolerance inducing-agent comprising the protease as an active ingredient.

As a result of intensive and extensive researches toward the solution of the above-mentioned problem, the present inventor has purified a novel SH-dependent protease from the placenta of a mammal and found that the protease has an excellent action of inducing immunological tolerance. Thus, the present invention has been achieved.

The present invention relates to a protease which is obtainable by crushing placentas of a mammal to thereby obtain a homogenate, centrifuging the homogenate and subjecting the resultant supernatant to affinity chromatography (I), ion exchange chromatography, gel filtration chromatography and affinity chromatography (II) in this order and which has the following physicochemical properties.

(1) Function

The protease catalyzes in the presence of β-mercaptoethanol the following enzyme reaction in which L-prolyl-L-phenylalanyl-L-arginine 4-methyl-coumaryl-7-amide (Pro-Phe-Arg-MCA) is hydrolyzed to L-prolyl-L-phenylalanyl-L-arginine and 7-amino-4-methyl-coumarin:

L-prolyl-L-phenylalanyl-L-arginine 4-methyl-coumaryl-7-

amide + $H_2O$ → L-prolyl-L-phenylalanyl-L-arginine +

7-amino-4-methyl-coumarin

(2) Optimum pH Range

The optimum pH is from 5.6 to 6.5 when Pro-Phe-Arg-MCA is used as a substrate.

(3) Range of Optimum Temperature

Appropriate action temperature ranges from 36.5 to 37.5°C.

(4) Stability

The purified protease in 5 mM acetate buffer (pH 5.0) completely retains its activity at -80°C for at least 30 days. The protease is completely denatured on heating at 100°C for 3 minutes.

(5) Molecular Weight

Its molecular weight is 30 kDa as determined by SDS-polyacrylamide gel electrophoresis followed by protein staining and by western blotting with biotin-labelled concanavalin A.

As a column to be used for the above affinity chromatography (I), Affi Gel 501 column may be given. As a column to be used for the above ion exchange chromatography, CM-Toyopearl 650$_M$ column may be given. As a column to be used for the above gel filtration chromatography, Toyopearl HW-50s column may be given. As a column to be used for the above affinity chromatography (II), Con A-Sepharose 4B column may be given.

The present invention also relates to an immunological tolerance inducing-agent comprising the protease described above as an active ingredient.

BRIEF DESCRIPTION OF THE DRAWINGS

Fig. 1 is a chart showing an immunosuppression protocol.
Fig. 2 is a graph showing the result that the protease of the invention suppresses mouse delayed footpad reaction.
Fig. 3 is a graph showing the result that the protease of the invention suppresses antibody production against sheep red blood cell.
Fig. 4 is a graph showing the preventive effects of the protease of the invention against mouse renal disorders.
Fig. 5 is a graph showing the results of the affinity chromatography of a crude extract of the protease of the invention using Affi Gel 501 column.
Fig. 6 is a graph showing the results of the cation exchange chromatography of the partially purified protease of the invention using CM-Toyopearl 650$_M$ column.
Fig. 7 is a graph showing the results of the gel filtration of fractions from the protease of the invention using Toyopearl HW-50s column.
Fig. 8 is a graph showing the results of the affinity chromatography of fractions from the protease of the invention using Con A-Sepharose 4B column.
Fig. 9 is a film copy of the SDS-polyacrylamide gel electrophoresis of the purified protease of the invention.

DETAILED DESCRIPTION OF THE INVENTION

Hereinbelow, the present invention will be described in detail.
The protease of the invention can be separated and purified by crushing placentae of a mammal to thereby obtain a homogenate, centrifuging the homogenate and subjecting the resultant supernatant to affinity chromatography (I), ion exchange chromatography, gel filtration chromatography and affinity chromatography (II) in this order.
First, several individuals of a mammal which is selected as the raw material are subjected to an abdominal operation to thereby remove their placenta. As a mammal, mouse, rat, rabbit, human and the like may be enumerated. The placenta tissue sections thus removed are crushed in a crusher to thereby prepare a homogenate. For the crushing of tissue sections, a conventional homogenizer such as a Potter-Elvehjem homogenizer may be used. As a buffer used for the crushing, for example, 20 mM Tris-HCl buffer (pH 7.2) containing 0.2% Triton X-100 and 0.15M sodium chloride may be preferable.
Subsequently, the resultant homogenate is centrifuged to thereby obtain a supernatant. As centrifugation, ultracentrifugation is preferable and it is carried out, for example, at 4 °C at 105,000xg for 60 minutes.
The resultant supernatant is subjected to chromatography to thereby purify the protease. The chromatography is composed of the 4 steps of affinity chromatography (I), ion exchange chromatography, gel filtration chromatography and affinity chromatography (II), and these steps are conducted in this order. Fractions obtained by each chromatography are subjected to appropriate desalting dialysis and concentration.
As a column for the affinity chromatography (I), for example, Affi Gel 501 column (Bio Rad) or a column packed with an affinity ligand or the like having a performance comparable to that of the former may be used. As a column for the ion exchange chromatography, for example, CM-Toyopearl 650$_M$ column (Tosoh Corp.) may be used. As a column for the gel filtration chromatography, for example, Toyopearl HW-50s column (Tosoh Corp.) or a column packed with a gel filtration matrix or the like having a separation performance comparable to that of the former may be used. As a column for the affinity chromatography (II), for example, Con A-Sepharose 4B column (Pharmacia) or a column packed with a concanavalin A-immobilized matrix or the like may be used.

The operation of each chromatography may be carried out according to the instructions of each manufacturer of matrix.

According to the procedures described above, finally, an SH-dependent protease is purified to homogeneous.

The resultant protease thus obtained has the following physicochemical properties.

(1) Function

The protease catalyzes in the presence of β-mercaptoethanol the following enzyme reaction in which L-prolyl-L-phenylalanyl-L-arginine 4-methyl-coumaryl-7-amide is hydrolyzed to produce L-prolyl-L-phenylalanyl-L-arginine and 7-amino-4-methyl-coumarin:

L-prolyl-L-phenylalanyl-L-arginine 4-methyl-coumaryl-7-

amide + $H_2O$ → L-prolyl-L-phenylalanyl-L-arginine +

7-amino-4-methyl-coumarin

(2) Optimum pH Range

The optimum pH is from 5.6 to 6.5 when Pro-Phe-Arg-MCA (MCA: 4-methyl-coumaryl-7-amide) is used as a substrate.

(3) Range of Optimum Temperature

Appropriate action temperature ranges from 36.5 to 37.5°C.

(4) Stability

The purified protease in 5 mM acetate buffer (pH 5.0) completely retains its activity at -80°C for at least 30 days. The protease is completely denatured on heating at 100°C for 3 minutes.

(5) Molecular Weight

Its molecular weight is 30 kDa as a result of determination by SDS-polyacrylamide gel electrophoresis followed by protein staining and by western blotting with biotin-labelled concanavalin A.

From what has been described so far, the purified protease product has been judged to be an SH-dependent protease.

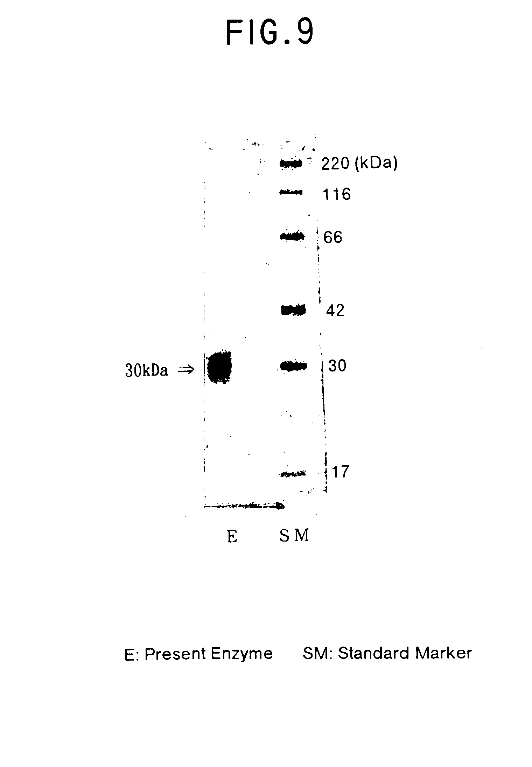

In order for the purified product to hydrolyze a peptide substrate (Pro-Phe-Arg-MCA) in 0.2M sodium acetate buffer (pH 6.0), the presence of the reducing agent such as β-mercaptoethanol is indispensable. Therefore, it is suggested that the protease of the invention (hereinafter referred to as the "present enzyme") is an SH-dependent protease. Furthermore, the present enzyme is detected as a single protein band of 30 kDa when subjected to SDS-polyacrylamide gel electrophoresis followed by either protein staining with Coomassie Blue (see Fig. 9) or silver staining.

In the detection of the present enzyme by western blotting, the enzyme has also been confirmed to exhibit a single band of 30 kDa when biotin-labelled concanavalin A is used.

These results suggest that the present enzyme is a glycoprotein containing mannose and/or glucose residue(s).

Now, the immunological tolerance inducing-agent of the invention will be described below.

When the present enzyme is used as an immunological tolerance inducing-agent, the object of administration is not particularly limited. The immunological tolerance inducing-agent of the invention may be used for the purpose of preventing or treating, for example, various allergic diseases (such as atopic dermatitis, bronchial asthma, allergic rhinitis, systemic lupus erythematosus, chronic articular rheumatism, etc.) and rejection resulted from organ transplantation (e.g., rejection of a graft in bone marrow, skin, heart, liver or kidney transplantation). The route of administration is parenteral and includes injections (e.g., intravenous injection, subcutaneous injection, intramuscular injection, drip, etc.), suppositories, creams, poultices and the like. The amount of administration varies depending on whether the subject is a human or an animal other than human, and the age of the subject, the route of administration and the number of times of administration, and the amount may be widely varied. When the present enzyme is administered as an immunological tolerance inducing-agent in the form of a composition comprising the present enzyme, an appropriate diluent and a pharmacologically acceptable carrier, the effective amount is 10 - 1000µg/kg/day and this amount may be administered once a day or may be divided into several times a day.

When the immunological tolerance inducing-agent of the invention is administered parenterally, the agent contains additives such as a stabilizer, a buffer, a preservative and an isotonicity inducing-agent, and is provided in the form of ampules each containing a unit dosage, a multidose container or a tube. The above-mentioned composition may also be a powder which is redissolved in an appropriate carrier, such as sterilized pyrogen-free water, before use.

Hereinbelow, pharmacological test examples will be described which support the fact that the present enzyme is useful as an immunological tolerance inducing-agent.

[Test Example 1]

Induction of Immunological Tolerance against Cellular Immune Reaction

First, a test concerning the suppression of cellular immune reaction by the present enzyme was conducted.

In order to immunize mouse, sheep red blood cells (SRBC) were administered. The day when the antigen was administered is designated "day 0". Six test groups were prepared which were scheduled to receive the administration of the present enzyme 15 days, 5 days, 4 days and 1 day before day 0, on day 0, and 1 day after day 0, respectively. Each administration group consisted of six C3H/He mice (female) of 12-week old. As a control group, mice which do not receive the administration of the present enzyme were used.

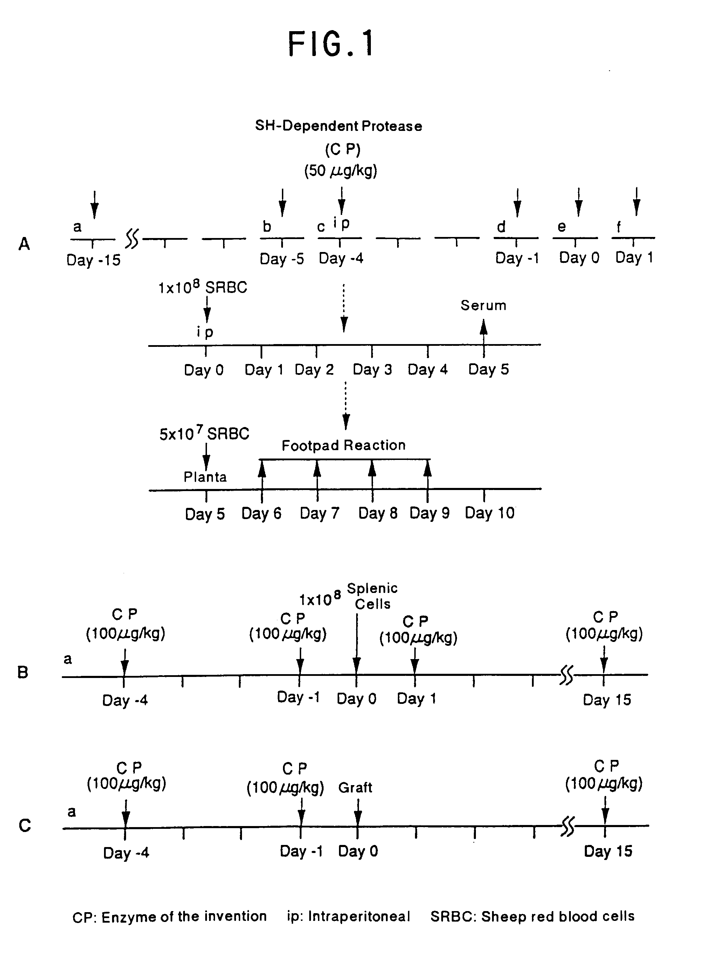

On each of the days as indicated above, the present enzyme (50 µg/kg) was intraperitoneally administered into the mice of each administration group (Fig. 1A, a-f). On day 0, immunization was carried out by intraperitoneally administering 0.1 ml of a saline containing SRBC ($1 \times 10^8$ cells), the antigen (Fig. 1A). With respect to the group which was scheduled to receive the present enzyme 1 day after day 0, the present enzyme was administered after the administration of SRBC. Subsequently, 5 days after day 0, 0.05 ml of a saline containing SRBC ($5 \times 10^7$ cells) was administered into each mouse at the right hind footpad to thereby induce delayed footpad reaction (DFR). The thickness of the footpad was measured for 4 days from the day after this administration (Fig. 1A). The term "Day 0" in Fig. 1 means the day when the first immunization was conducted. Negative figures appearing in Fig. 1 represent the number of days before the first immunization (e.g., "Day -1" means 1 day before the immunization) and positive figures therein represent the number of days after the first immunization (e.g., "Day 1" means 1 day after the immunization).

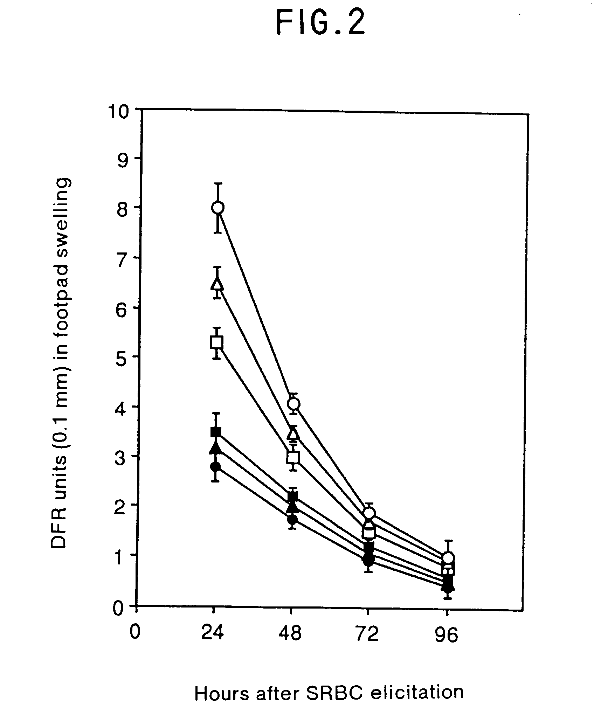

As a result, the footpad reaction (swelling) was significantly suppressed (t-test, $P < 0.01$) in the three groups which were administered the present enzyme 1 day before the intraperitoneal administration of the antigen, on day 0, and a day after the intraperitoneal administration of the antigen, respectively, compared to the control group (Fig. 2). This suggests that the administration of the present enzyme suppresses the delayed hypersensitive reaction which is the cellular immune against SRBC. In Fig. 2, mark "Δ" represents the group which received the present enzyme 15 days before the administration of the antigen; mark "□" represents the groups which received the present enzyme 5 days or 4 days before the administration of the antigen; mark "▲" represents the group which received the present enzyme 1 day before the administration of the antigen; mark "●" represents the group which received the present enzyme on day 0 (on the day of the antigen administration); mark "■" represents the group which received the present enzyme 1 day after the administration of the antigen; and mark "○" represents the control group. The results are shown as the mean value of 6 mice± standard deviation.

Subsequently, using the group which received the present enzyme 1 day before the first administration of the antigen, the second immunization was carried out three months after the first administration of the antigen (i.e., three months after day 0). Like in the first immunization, the antigen was 0.1 ml of a saline containing SRBC ($1 \times 10^8$ cells), and this was administered intraperitoneally. Five days after this administration, 0.05 ml of a physiological saline containing $5 \times 10^7$ SRBCs was injected in order to induce footpad reaction (swelling) in each mouse. On the following day (24 hours after the injection), the swelling (thickness) in the footpad was measured. Additionally, the group which received the present enzyme 4 days before the first administration of the antigen and the control group were treated similarly as described above, and the footpad swelling was measured.

As a result, when the second administration of the antigen was carried out, it was observed that the suppressive effects upon the immune reaction to SRBC was lasting in the group which received the present enzyme 1 day before the first administration of the antigen, even though this group was not newly received the present enzyme 1 day before the second administration of the antigen. Thus, it has become clear that the present enzyme induces immunological tolerance (Table 1).

Table 1. Effects of the Present Enzyme upon Footpad Reaction and Humoral Antibody Production against SRBC in Mice

| Treatment with Enzyme | Immunization | Extent of Footpad Reaction (Thickness of swelling; unit= 0.1 mm) 24 hours after immunization | P Value (t-test) | Antibody Titer (Log$_2$); 5 days after immuniza. | P Value (t-test) |
|---|---|---|---|---|---|
| Untreated Group | 1st immunization | 6.5 ± 0.2 | – | 6.0 ± 0.2 | – |
| | 2nd immunization | 7.0 ± 0.2 | – | 7.0 ± 0.2 | – |
| Group treated with the present enzyme[3] (1 day before the 1st immunization) | 1st immunization[1] | 2.5 ± 0.2 | <0.01 | 5.5 ± 0.2 | NS[4] |
| | 2nd immunization[2] | 3.5 ± 0.2 | <0.01 | 6.5 ± 0.2 | NS |
| Group treated with the present enzyme (4 days before the 1st immunization) | 1st immunization | 6.0 ± 0.2 | NS[4] | 4.0 ± 0.2 | <0.05 |
| | 2nd immunization | 7.0 ± 0.2 | NS | 5.0 ± 0.2 | <0.05 |

1) In the first immunization, SRBC was administered after the administration of the present enzyme.

2) In the second immunization, those mice which had undergone the first immunization were administered SRBC alone as an antigen 3 months after the 1st immunization.

3) Placenta protease derived from C3H ♀ x C3H♂.

4) No significant difference.

EP 0 771 869 A1

[Test Example 2]

Induction of Immunological Tolerance against Humoral Antibody Production

A test concerning the suppression of humoral immune reaction by the present enzyme was carried out.

The immunization of mice, the schedule of administration and the dose of the present enzyme, and the use of test groups and control group were the same as described in Test Example 1.

Five days after the first immunization, blood was collected from mice in each group and the antibody titer in serum (HA) against SRBC was determined. The determination of the antibody titer was conducted according to a conventional method [Sever, J. Immunology, 88:320, (1962)] using hemagglutination by the microtiter method with SRBC as an antigen.

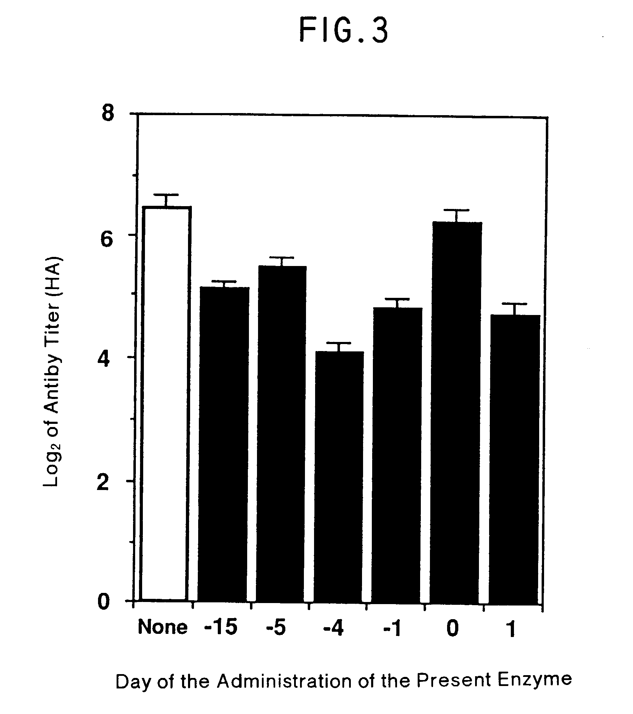

As a result, in the group which was administered the present enzyme 4 days before the immunization, the antibody production was significantly suppressed (t-test, $P<0.05$) compared to the control group (Fig. 3). Thus, it was found that the administration of the present enzyme suppresses the production of humoral antibodies against SRBC. The terms "-15", "-5", "-4", "-1", "0" and "1" appearing in Fig. 3 represent the day when the present enzyme was administered. They mean 15 days, 5 days, 4 days and 1 day before the first immunization, on the day of the first immunization, and 1 day after the first immunization, respectively. The results are shown as the mean value of 6 mice ± standard error.

Furthermore, when the second immunization was conducted 3 months after the first immunization, it was also observed that the suppressive effects upon the immune reaction to SRBC was lasting in the group which received the present enzyme 4 days before the first immunization even though the group was not newly administered the present enzyme, as described previously (Table 1). Thus, it has become clear that the present enzyme induces immunological tolerance.

[Test Example 3]

Comparison of Immunosuppressions Induced by the Present Enzyme and Other Proteases

A comparative test was conducted to compare immunosuppressions induced by the present enzyme and other protease. As other proteases, trypsin, plasmin, collagenase and cysteine protease were used, and an untreated group was used as a control group.

Each of these proteases (50 µg/kg) was intraperitoneally administered to 12-week old C3H/He mice (♀). One day after the administration, the mice were immunized by intraperitoneally injecting 0.1 ml of a saline containing SRBC ($1\times10^8$ cells) as an antigen. Further, 5 days after the immunization, 0.05 ml of a saline containing SRBC ($5\times10^7$ cells) was injected to mice at the footpad and the footpad swelling (thickness) against SRBC was measured.

As a result, in the groups which were administered the present enzyme or other cysteine protease 1 day before the administration of the antigen, the footpad reaction (swelling) was significantly suppressed (t-test, $P<0.01$) compared to the control group (Table 2). Thus, it was shown that the administration of either the present enzyme or other protease induces the suppression of delayed hypersensitive reaction which is the cellular immune against SRBC. However, the administration of any of the proteases of trypsin, plasmin and collagenase which are not cysteine protease family members did not exhibit significant difference from the control group, and immunosuppression was not induced by such proteases (Table 2).

Table 2. Footpad Reaction and Antibody Production in Mice Immunized with SRBC after Administration of Various Proteases

| Treatment with Enzyme | Origin | Extent of Footpad Reaction (Thickness of swelling; unit = 0.1 mm) 24 hours after immunization | P Value (t-test) | Antibody Titer ($Log_2$); 5 days after immuniza. | P Value (t-test) |
|---|---|---|---|---|---|
| Untreated | | 8.0 ± 0.5 | – | 6.5 ± 0.3 | – |
| Trypsin | Porcine spleen | 6.0 ± 0.4 | NS[5] | 6.0 ± 0.3 | NS |
| Plasmin | Porcine blood | 6.0 ± 0.3 | NS | 6.0 ± 0.3 | NS |
| Collagenase | Clostridium [1] | 6.0 ± 0.4 | NS | 6.0 ± 0.3 | NS |
| Cysteine protease | Metacercaria [2] | 3.0 ± 0.3 | <0.01 | 4.0 ± 0.3 | <0.05 |
| Cysteine protease | Plerocercoid [3] | 3.0 ± 0.3 | <0.01 | 3.5 ± 0.2 | <0.05 |
| Present Enzyme | Mouse placenta [4] | 3.0 ± 0.2 | <0.01 | 3.5 ± 0.2 | <0.05 |

1) *Clostridium histolyticum*

2) *Paragonimus westermani*

3) *Spirometra erinancei*

4) Placenta derived from C3H♀ x C3H♂.

5) No significant difference.

EP 0 771 869 A1

Subsequently, a comparative test was conducted to examine the suppression of the production of humoral antibodies against SRBC by these proteases.

Each protease (50 µg/kg) was intraperitoneally administered to mice 15 days and 4 days before the administration of the antigen. Then, on day 0 mice were immunized by intraperitoneally administering 0.1 ml of a saline containing SRBC ($1 \times 10^8$ cells). Five days after the immunization, the antibody titer in serum (HA) against SRBC was determined for mice in each group. The method for determination of the antibody titer was as described previously.

As a result, in groups which were administered the present enzyme and other cycteine protease, the antibody production was significantly suppressed (t-test, $P<0.05$) compared to the control group (Table 2). It has been made clear that either the administration of the present enzyme or the administration of other cysteine protease suppresses the production of humoral antibodies against SRBC. However, in any of the groups which were administered trypsin, plasmin or collagenase, no significant difference from the control group was observed, and thus immunosuppression was not induced (Table 2).

[Test Example 4]

Suppression of the Incidence of Autoimmune Diseases

The present enzyme (100 µg/kg for each time) was administered intraperitoneally to 7-week old MRL-lpr/lpr mice (♀) 4 days and 1 day before the administration of an antigen. On day 0, splenic cells ($1 \times 10^8$) from 22-week old MRL-lpr/lpr mice (♀) were intraperitoneally administered as an antigen. Further, 1 day and 15 days after the administration of the antigen, the present enzyme (100 µg/kg for each time) was administered intraperitoneally (Fig. 1B).

After the administration of the antigen, the survival of mice were examined until they reached 21-week old. Using 22-week old mice, renal disorder scores were determined which would serve as indexes for autoimmune diseases.

The determination of renal disorder scores was conducted as follows.

First, each kidney was fixed in 10% formalin and paraffin sections were prepared. These sections were stained with hematoxylin and eosin to thereby obtain preparations. Then, 20 glomeruli of renal tissues in each of the preparations were microscopically examined, and microscopic images exhibiting nephritis were given the following points. Normal glomerulus: 1 point; multiplicate nuclear image: 2 points; proliferative nephritis: 3 points; glassy body formation: 4 points and crescent formation: 5 points. The number of each image was multiplied by the appropriate point to thereby calculate the score of each preparation. Thus, mean nephritis score for each group was obtained.

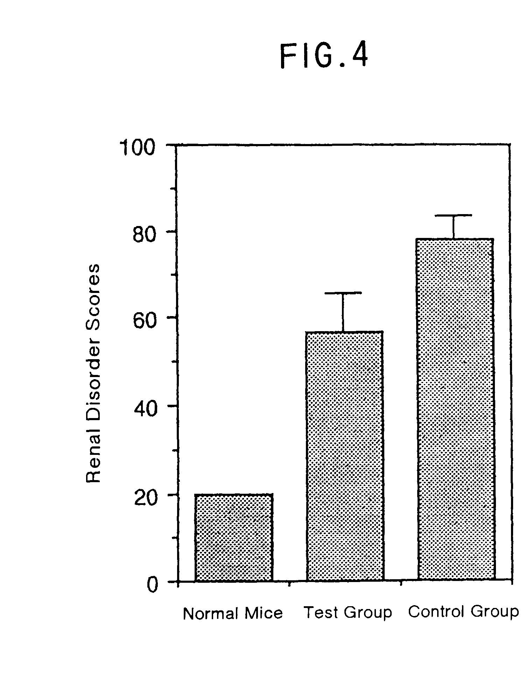

As a result, the in test groups which were administered the present enzyme, the incidence of autoimmune diseases in MRL-lpr/lpr mice (i.e., the mortality of 21-week old mice and the renal disorders of 22-week old mice) could be suppressed and immunological tolerance could be induced [Table 3: chi-square test ($P<0.01$); Fig. 4: t-test ($P<0.05$)]. The P values were based on comparison between the test groups and the control group.

Table 3.

| Effects of the Present Enzyme upon Mouse Survival Ratio (at 21-Week Old) | | | |
|---|---|---|---|
| Treatment with Enzyme | Antigen | Mortality (Decrease ratio) | P Value (Chi-square test) |
| Not treated | - | 5/10 (50%) | - |
| Present enzyme[1] | - | 4/10 (40%) | NS[3] |
| - | Splenic cells[2] | 3/10 (30%) | NS |
| Present enzyme | Splenic cells | 0/10 (0%) | <0.01 |

1) Protease derived from placentae from C3H♀ xC3H♂.

2) Splenic cells from MRL-lpr/lpr (♀ ; 22-week old).

3) No significant difference.

[Test Example 5]

Test for Inducing Immunological Tolerance Leading to Prolonged Skin Take

The effects of immunological tolerance induced by the present enzyme upon skin take when skin grafts from AKR mice (♀) were transplanted to C3H/He mice (♀) was tested.

Four days and 1 day before the transplantation of skin, the present enzyme (100 µg/kg for each time) was intraperitoneally injected to 8-week old C3H/He mice (♀), to which skin grafts from 6-week old AKR mice (♀) were transplanted (Fig. 1C). Fifteen days after the skin transplantation, the present enzyme (100 µg/kg) was administered and then the period of survival of a skin graft in each mouse was measured. Additionally, a group which was not administered the present enzyme was used as a control group.

As a result, while the mean survival time (MST) of a skin graft was 17.5 ± 0.4 days in the control group, the MST in the test group was 41.7 ± 10.3 days. Thus, the present enzyme could achieve skin take for a significantly longer period of time (t-test, $P < 0.05$) (Table 4).

Table 4. Effects of the Present Enzyme upon the Survival of Mouse Skin Grafts

| Recipient Mouse | Skin Graft Donor Mouse | Schedule for Administration of Present Enzyme[1] ($\mu$ g/kg) | | | Period of Survial of Skin Graft in Each Mouse (Days) | Mean Survival Time[2] | P Value (t-test) |
|---|---|---|---|---|---|---|---|
| | | 4 days before | 1 day before | 15 days after | | | |
| C3H(♀) | AKR(♀) | – | – | – | 16, 17, 17, 18, 18, 19 | 17.5±0.4 | – |
| C3H(♀) | AKR(♀) | 100 | 100 | 100 | 23, 23, 24, 30, 60, 90 | 41.7±10.3 | <0.05 |

1) Protease derived from placentas from C3H(♀) x AKR(♂).

2) Days ± Standard Error

EP 0 771 869 A1

These results shows that the intraperitoneal administration of the present enzyme to mouse induces immunological tolerance against antigens and tissues injected or transplanted before or after the above administration.

From the Test Examples described above, it was confirmed that the administration of the present enzyme suppresses the production of antibodies against antigens or transplanted tissued and induces immunological tolerance in organisms.

In each of the above Test Examples, an SH-dependent protease directly extracted from mammal placentae was used. However, when the present invention is practiced in a commercial size, there may be used an SH-dependent protease which is prepared in large quantities from cultured animal cells or, by using genetic engineering techniques (recombinant DNA techniques), from transformants.

PREFERRED EMBODIMENTS OF THE INVENTION

Hereinbelow, the present invention will be described more specifically with reference to the following Examples. However, the present invention is not limited to these Examples.

EXAMPLE 1

Purification of a Mammal-Derived SH-Dependent Protease

Six placentae (about 5 g) from C3H mice were crushed in 20 mM Tris-HCl buffer(pH 7.2) containing 0.2% Triton X-100 and 0.15 M NaCl (hereinafter referred to as "TBS-7.2"), and 20 ml of the resultant homogenate was centrifuged at 4°C for 1 hour at 105,000xg.

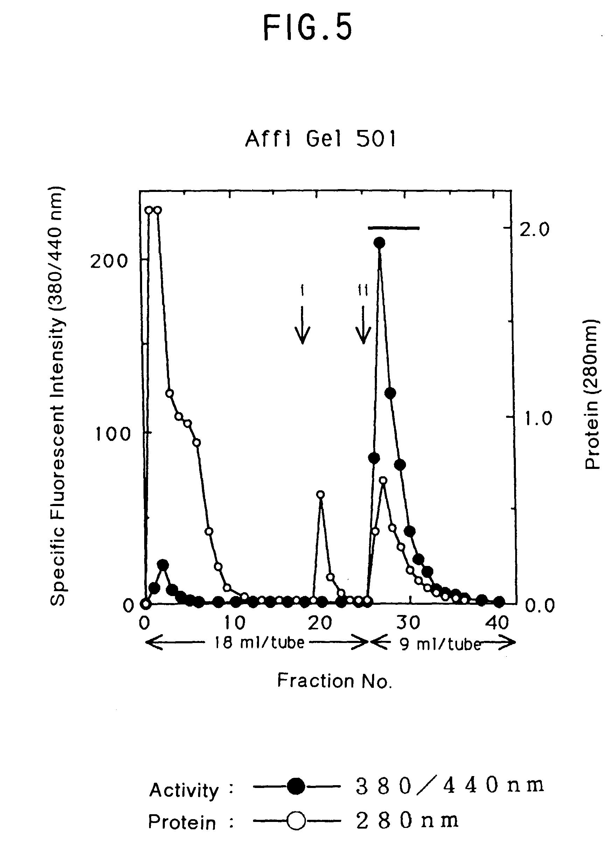

Then, a crude extract of the resultant supernatant was adsorbed on an Affi Gel 501 column (2.5 x 3.8 cm; Bio Rad) which had been equilibrated with TBS-7.2. Then, the column was washed with 10 column volumes of TBS-7.2 to thereby remove unadsorbed proteins from 5 the column. Further, those proteins which were adsorbed on the column with weak affinity interaction were removed by washing with TBS-7.2 containing 40 mM EDTA (disodium ethylenediamine tetraacetate). Thereafter, an SH-containing proteins specifically binding to the column was eluted with TBS-7.2 containing 10 mM β-mercaptoethanol and 40 mM EDTA (Fig. 5).

The results of the above elution are shown in Fig. 5. Those active fractions indicated with a mark "—" in Fig . 5 were collected (i.e., fractions Nos. 26 - 31).

At the time point indicated with the arrow marked with "i" in Fig. 5, non-enzyme proteins weakly bound to the column were eluted and removed with EDTA. Then, at the time point indicated with the arrow marked with "ii", SH proteins having an activity were eluted with EDTA and β-mercaptoethanol.

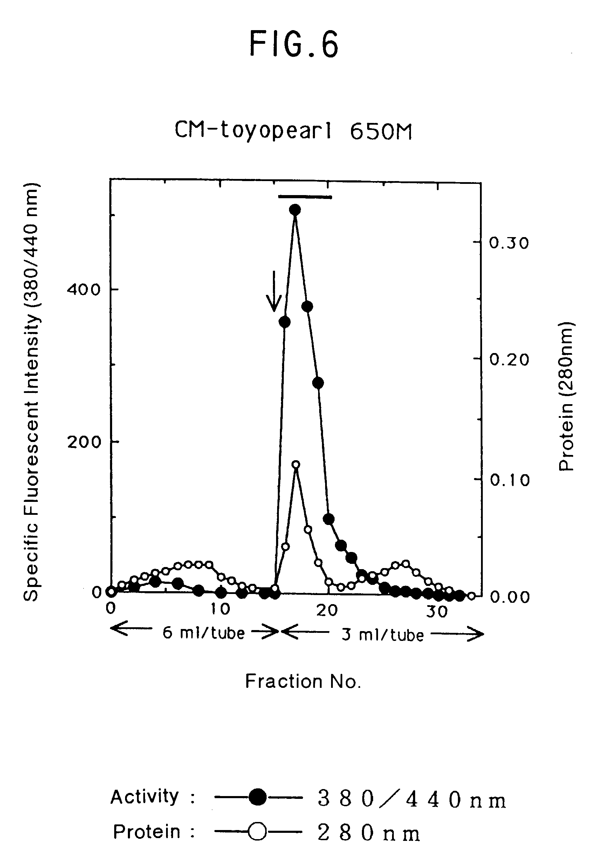

The partially purified enzyme solution collected from the affinity chromatography using an Affi Gel 501 column (i. e., the portion indicated with a mark "—" in Fig. 5; fractions Nos. 26 - 31) was dialyzed against 20 mM acetate buffer, pH 4.6 (hereinafter referred to as "AB-4.6") for 12 hours. The dialyzed enzyme fraction solution was adsorbed on a CM-Toyopearl 650M column which is a cation exchanger equilibrated with AB-4.6, and unadsorbed proteins were removed by washing with AB-4.6. Adsorbed proteins were eluted from the column with AB-4.6 containing 75 mM NaCl (Fig. 6).

In Fig. 6, first, non-enzyme proteins to the cation exchanger were removed by washing. Then, from the time point indicated with an arrow "↓", active fractions were eluted. Here, the fractions indicated with a mark "—" (i.e., fractions Nos. 16 - 20) were collected as a partially purified enzyme.

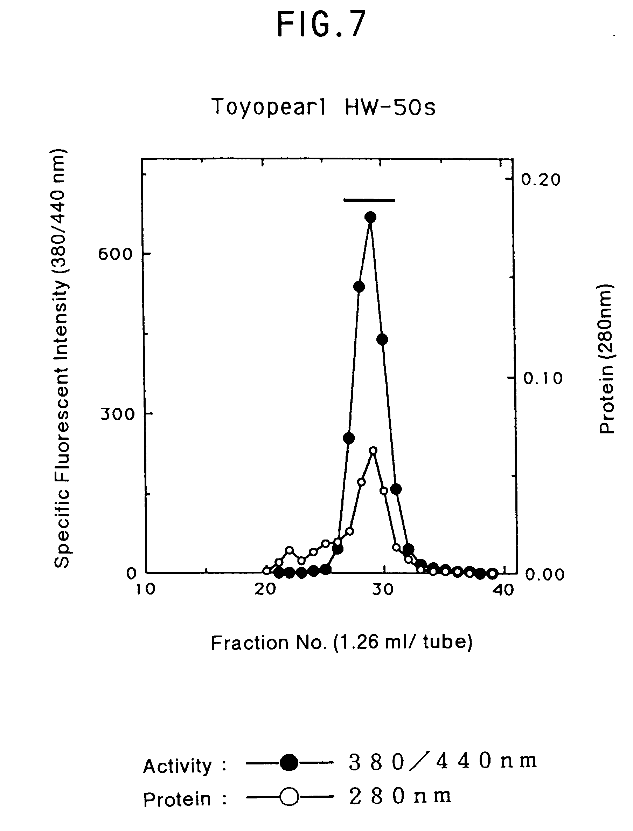

This enzyme solution obtained from the ion exchange chromatography using a CM-Toyopearl column was concentrated to 1 ml by ultrafiltration. This concentrated enzyme solution was applied to a Toyopearl HW-50s column which had been equilibrated with 20 mM acetate buffer, pH 5.0 containing 0.15M-NaCl (hereinafter referred to as "ABS-5.0") to thereby conduct gel filtration chromatography (Fig. 7).

Of those fractions showing activity in Fig. 7, the active portions indicated with a mark "—" (i.e., fractions Nos. 27 - 31) were collected so that contaminant proteins were removed.

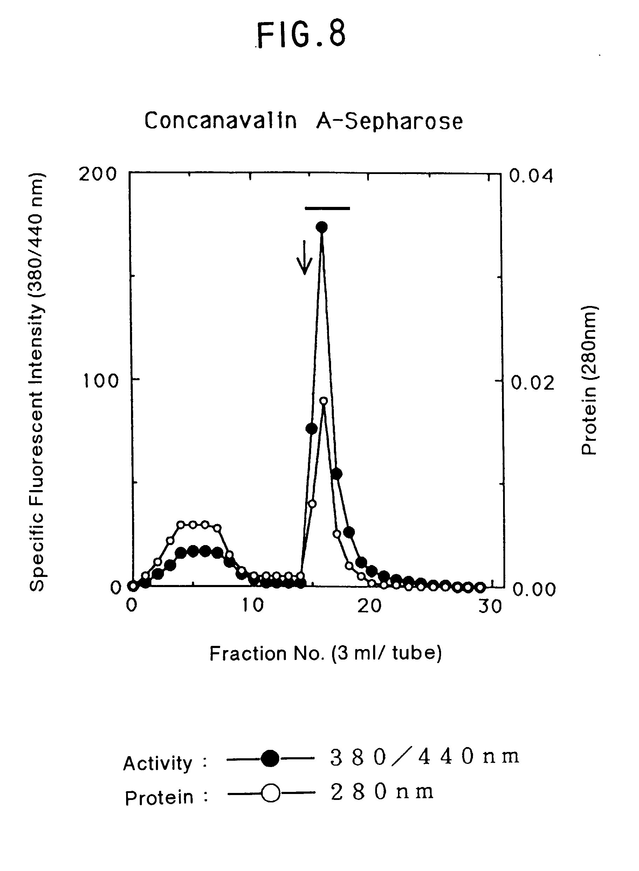

Subsequently, the enzyme was eluted from the gel chromatography column with ABS-5.0. The resultant eluate having an enzyme activity was subjected to affinity chromatography using a Con A-Sepharose 4B (1.5 x 3.5 cm; Pharmacia) equilibrated with ABS-5.0 containing 1 mM CaCl$_2$ and 1 mM MnCl$_2$. First, unadsorbed proteins in the column were removed by washing and then the enzyme of interest was specifically eluted with 0.1 M methyl mannose in 0.2 M Tris-HCl (pH 7.2) containing 1 mM CaCl$_2$ and 1 mM MnCl$_2$ (Fig. 8).

After contaminant proteins exhibiting no affinity to concanavalin A were removed by washing, the enzyme of interest was specifically eluted with 0.1 M methyl mannose from the time point indicated with an arrow "↓". Those fractions indicated with a mark "—" (i.e., fractions Nos. 15 - 18) were collected.

Twelve milliliters of the eluate [purified enzyme (150 µg protein)] was dialyzed against 5mM acetate buffer (pH 5.0) at 4 °C for 6 to 9 hours and concentrated to 0.2 ml by ultrafiltration to thereby obtain the purified enzyme.

The enzyme thus purified was confirmed as a single protein band exhibiting a molecular weight of 30 kDa as a result of electrophoresis (protein staining) (Fig. 9).

EFFECT OF THE INVENTION

According to the present invention, a novel SH-dependent protease and an immunological tolerance inducing-agent comprising the protease as an active ingredient are provided.

When administered to a mammal several times, the immunological tolerance inducing-agent of the invention can suppress the production of antibodies against antigens and transplanted tissues and induce immunological tolerance. Therefore, the immunological tolerance inducing-agent of the invention is free from the necessity of a long-term administration which is the disadvantage of conventional immunosuppressants. Thus, the immunological tolerance inducing-agent of the invention produces an excellent effect that fear for side effects can be eliminated.

**Claims**

1. An SH-dependent protease which is obtainable by crushing placentae of a mammal to thereby obtain a homogenate, centrifuging the homogenate and subjecting the resultant supernatant to affinity chromatography (I), ion exchange chromatography, gel filtration chromatography and affinity chromatography (II) in this order and which has the following physicochemical properties:

   (i) Function: catalyzing in the presence of β-mercaptoethanol the following enzyme reaction in which L-prolyl-L-phenylalanyl-L-arginine 4-methyl-coumaryl-7-amide is hydrolyzed to produce L-prolyl-L-phenylalanyl-L-arginine and 7-amino-4-methyl-coumarin:

   L-prolyl-L-phenylalanyl-L-arginine 4-methyl-coumaryl-7-amide

   $+ H_2O \rightarrow$ L-prolyl-L-phenylalanyl-L-arginine + 7-amino-4-

   methyl-coumarin

   (ii) Optimum pH Range: from pH 5.6 to 6.5 when Pro-Phe-Arg-MCA is used as a substrate.

   (iii) Range of Optimum Temperature: from 36.5 to 37.5 °C.

   (iv) Stability: when purified and stored in 5 mM acetate buffer (pH 5.0), completely retaining its activity at -80°C for at least 30 days. Being completely denatured on heating at 100°C for 3 minutes.

   (v) Molecular Weight: 30 kDa as determined by SDS-Polyacrylamide gel electrophoresis followed by protein staining and by western blotting with biotin-labelled concanavalin A.

2. The SH-dependent protease of claim 1, wherein the affinity chromatography (I) is carried out using an Affi Gel 501 column.

3. The SH-dependent protease of claim 1 or 2, wherein the ion exchange chromatography is carried out using a CM-Toyopearl 650$_M$ column.

4. The SH-dependent protease of any one of claims 1 to 3, wherein the gel filtration chromatography is carried out using a Toyopearl HW-50s column.

5. The SH-dependent protease of any one of claims 1 to 4, wherein the affinity chromatography (II) is carried out using a Con A-Sepharose 4B column.

6. An immunological tolerance inducing-agent comprising as an active ingredient the SH-dependent protease of any one of claims 1 to 5.

7. A pharmaceutical composition comprising the SH-dependent protease of any one of claims 1 to 5 together with a diluent and pharmacologically acceptable carrier.

8. The SH-dependent protease of any one of claims 1 to 5 or the composition of claim 7 for use in a method of treatment of the human or animal body.

9. The SH-dependent protease of any one of claims 1 to 5 or the composition of claim 7 for use in a method of treatment of an allergic disease or rejection resulting from organ transplantation in the human or animal body.

# FIG.1

SH-Dependent Protease
(C P)
(50 μg/kg)

A    a   b   c ip   d   e   f

Day -15   Day -5   Day -4   Day -1   Day 0   Day 1

1x10⁸ SRBC

ip   Serum

Day 0   Day 1   Day 2   Day 3   Day 4   Day 5

5x10⁷ SRBC

Footpad Reaction

Planta

Day 5   Day 6   Day 7   Day 8   Day 9   Day 10

1x10⁸ Splenic Cells

B   a   C P (100μg/kg)   C P (100μg/kg)   C P (100μg/kg)   C P (100μg/kg)

Day -4   Day -1   Day 0   Day 1   Day 15

C   a   C P (100μg/kg)   C P (100μg/kg)   Graft   C P (100μg/kg)

Day -4   Day -1   Day 0   Day 15

CP: Enzyme of the invention    ip: Intraperitoneal    SRBC: Sheep red blood cells

# FIG.2

Hours after SRBC elicitation

# FIG.3

Day of the Administration of the Present Enzyme

# FIG.4

# FIG.5

Affi Gel 501

# FIG.6

CM-toyopearl 650M

Activity :  ——●——  380／440 nm
Protein :   ——○——  280 nm

# FIG.7

Toyopearl HW-50s

Activity : ——●—— 380／440 nm
Protein : ——○—— 280 nm

EP 0 771 869 A1

# FIG.8

## Concanavalin A-Sepharose

Activity : ——●—— 380／440 nm
Protein : ——○—— 280 nm

22

EP 0 771 869 A1

# FIG.9

220 (kDa)
116
66
42
30kDa ⇒    30
17

E    S M

E: Present Enzyme    SM: Standard Marker

23

**European Patent Office**

# EUROPEAN SEARCH REPORT

Application Number

EP 96 30 7916

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | PARASITE IMMUNOLOGY, vol. 16, 1994, OXFORD, GB, pages 261-273, XP000196694 F. HAMAJIMA ET AL.: "Immunosuppression by a neutral thiol protease from parasitic helminth larvae in mice" * the whole document * | 1-9 | C12N9/64 |
| Y | EP 0 524 834 A (F. HAMAJIMA ET AL.) 27 January 1993 * the whole document * | 1-9 | |
| Y | ACTA BIOCHIMICA POLONICA, vol. 36, no. 3-4, 1989, VARSAW, PL, pages 343-351, XP000197018 G. SAWICKI ET M. WARWAS: "Cathepsin H from human placenta" * the whole document * | 1-5 | |
| Y | PLACENTA, vol. 3, no. 1, 1982, LONDON,GB, pages 45-56, XP000197016 S.F. CONTRACTOR ET AL.: "Purification and properties of human placental cathepsin D" * the whole document * | 1-5 | **TECHNICAL FIELDS SEARCHED** (Int.Cl.6) C12N |
| Y | PLACENTA, vol. 7, no. 1, 1986, LONDON,GB, pages 73-80, XP000197015 N. RABBANI ET AL.: "Calcium-activated neutral protease from human placenta: purification and characterization" * the whole document * | 1-5 | |

-/--

The present search report has been drawn up for all claims

| Place of search | Date of completion of the search | Examiner |
|---|---|---|
| BERLIN | 19 February 1997 | Mateo Rosell, A.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**European Patent Office**

Application Number

EP 96 30 7916

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EUROPEAN JOURNAL OF BIOCHEMISTRY, vol. 83, no. 1, 1978, BERLIN, DE, pages 87-97, XP000197017 P. EVANS AND D.J. ETHERINGTON: "Characterisation of cathepsin B and collagenolytic cathepsin from human placenta" * the whole document * | 1-5 | |
| P,Y | DATABASE WPI Week 9642 Derwent Publications Ltd., London, GB; AN 96-425214 XP002025619 & WO 96 27381 A (F. HAMAJIMA AND K. YAMAKAMI) , 12 September 1996 * abstract * | 1-9 | |

**TECHNICAL FIELDS SEARCHED (Int.Cl.6)**

The present search report has been drawn up for all claims

| Place of search | Date of completion of the search | Examiner |
|---|---|---|
| BERLIN | 19 February 1997 | Mateo Rosell, A.M. |